# EUROPÄISCHE PATENTANMELDUNG

(11) **EP 0 702 022 A1**
(43) Veröffentlichungstag der Anmeldung: **20.03.1996**
(21) Anmeldenummer: 95110918.0
(22) Anmeldetag: 12.07.1995
(51) Int. Cl.: C07H 15/04

(54) **Verfahren zur Herstellung von Alkylglycosiden mit niedrigem Glycosidierungsgrad**

(30) Priorität: 07.09.1994 DE 4431854
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

In einem Verfahren zur Herstellung von Alkylglycosiden mit C₈- bis C₂₀-Alkylresten duch Glycosidierung und Umglycosidierung oder durch Direktglycosidierung geht man von einem Saccharidsirup mit einem Dextroseäquivalent von 90 bis 95 aus. Produkte mit sehr niedrigem mittlerem Glycosidierungsgrad werden dadurch erhalten, daß man während der Reaktion und/oder danach Oligosaccharid abfiltriert.

## Beschreibung

Die Erfindung betrifft ein neues ein- oder zweistufiges Verfahren zur Herstellung von Alkylglycosiden mit C₈- bis C₂₀-Alkylresten und mit einem mittleren Glycosidierungsgrad zwischen 1,05 und 1,4.

Alkylglycoside mit C₈- bis C₂₀-Alkylgruppen können ganz oder teilweise aus nachwachsenden Rohstoffen hergestellt werden. Sie gewinnen deshalb und auch wegen ihrer sehr guten biologischen Abbaubarkeit zunehmend an Bedeutung. Die Produkte haben neben ihren interessanten Tensideigenschaften den Vorteil, daß man ihre Polarität über die Länge der Alkylkette und über den Glycosidierungsgrad genau einstellen kann. Dadurch können die Alkylglycoside auf ihr Anwendungsgebiet gezielt ausgerichtet werden.

In EP-A-0 495 174 wird eine Direktglycosidierung von Sacchariden mit C₁₂- bis C₂₀-Alkoholen beschrieben. Bei dieser einstufigen Herstellung von Alkylpolyglycosiden wird Natriumhypophosphit zur Farbverbesserung zugesetzt. Man erhält hier Produkte mit mittleren Glycosidierungsgraden von 1 bis 8. Das Herstellverfahren ist jedoch auf Ausgangsmischungen mit einem sehr geringen Wassergehalt begrenzt. Es kann deshalb nicht mit Hilfe eines preiswerten Sirups durchgeführt werden.

Bei der zweistufigen Alkylglycosidherstellung wird zunächst eine Glycosidierung durchgeführt. Aus Sacchariden und C₂- bis C₆-Alkoholen werden dabei Alkylglycoside mit kurzkettigen Alkylgruppen hergestellt. Diese Produkte werden dann in der zweiten Stufe durch Umglycosidierung mit C₈- bis C₂₀-Alkoholen in die gewünschten Alkylglycoside mit Tensideigenschaften übergeführt.

Dieser Weg der Herstellung ist lange bekannt. Neuere Anmeldungen auf diesem Gebiet befassen sich häufig mit der Herstellung farblich verbesserter Produkte. Dabei werden zum Teil Reduktionsmittel zugesetzt oder spezielle Apparaturen verwendet.

So wird in EP-A-0 501 032 die Glycosidierung in einem Verdampfer vorgenommen. Der dort vorzugsweise verwendete Glucosesirup ist im allgemeinen ein Stärkehydrolysat mit Oligosaccharidanteilen. Das Dextroseäquivalent derartiger Sirupe liegt meist bei 90 bis 95. Die hier hergestellten Alkylpolyglycoside mit langkettigen Alkylgruppen weisen vorzugsweise mittlere Glycosidierungsgrade im Bereich von 1,3 bis 5 auf, wobei im Beispiel ein Wert von 1,65 angegeben wird.

Nach EP-A-0 514 628 kann auch die Umglycosidierung in einem Verdampfer vorgenommen werden. In EP-A-0 514 627 wird die Umglycosidierung in einer Reaktionskolonne durchgeführt. Dabei stellt man ein Molverhältnis von Alkylglycosid mit kurzem Alkylrest zu langkettigem Alkohol von 1 : 2 bis 1 : 15 ein und erhält Alkylpolyglycoside mit Glycosidierungsgraden von vorzugsweise 1,2 bis 3.

WO 93/101 33 beschreibt ein zweistufiges Verfahren zur Herstellung von Alkyloligoglycosiden, das kontinuierlich oder diskontinuierlich durchgeführt werden kann und bei dem ein Glucosesirup mit einem Anteil an monomerer Glucose von 90 bis 100 % eingesetzt wird. Das hier beschriebene Verfahren ist insgesamt recht aufwendig. Es erfordert sowohl bei der Glycosidierung als auch bei der Umglycosidierung eine Nachreaktion. Außerdem müssen in beiden Reaktionsstufen enge Temperaturbereiche eingehalten werden. WO 93/101 33 liefert dabei Alkyloligoglucoside mit einem Polyglucosegehalt von unter 5 Gewichtsprozent.

Zur destillativen Abtrennung der Fettalkohole von Alkylpolyglycosiden mit C₁₀- bis C₁₈-Alkylgruppen und einem mittleren Glycosidierungsgrad von 1,05 bis 1,4 verwendet man gemäß DE-A-41 29 587 einen Dünnschichtverdampfer. Auf die Herstellung der Alkylpolyglycoside wird hier nicht näher eingegangen.

Einen Hinweis darauf, wie Alkylglycoside mit mittleren Glycosiderungsgraden zwischen 1,05 und 1,4 in einfacher Weise hergestellt werden können, liefern die genannten Schriften nicht.

Es war daher Aufgabe der vorliegenden Erfindung, ein einfaches und preiswertes Verfahren zur Herstellung von Alkylglycosiden mit C₈- bis C₂₀-Alkylresten und mit einem mittleren Glycosidierungsgrad zwischen 1,05 und 1,4 bereitzustellen. Die Produkte sollten darüber hinaus auch eine gute Farbqualität aufweisen.

Die Aufgabe wird erfindungsgemäß dadurch gelöst, daß man einen Saccharidsirup mit einem Dextroseäquivalent von 90 bis 95 einsetzt und außerdem während und/oder nach der Reaktion Oligosaccharid abfiltert.

Überraschenderweise gelingt es durch diese Maßnahmen, beispielsweise in einfachen Rührkesselkaskaden Produkte von hoher farblicher Qualität mit niedrigem Glycosidierungsgrad herzustellen.

Ohne die Filtration treten praktisch unvermeidlich feste, unlösliche Bestandteile in den alkoholischen Reaktionsgemischen auf, die aus teilweise alkylierten Zuckeroligomeren bestehen und Rohrleitungen und Reaktoren verstopfen. Die Abtrennung dieser Oligosaccharide hat überraschenderweise eine starke Auswirkung auf den mittleren Glycosidierungsgrad.

Vorzugsweise erfolgt die Filtration mit Hilfe von Filtern mit Porengrößen von 50 bis 200 µm. Dabei werden Filter mit Porengrößen von 80 bis 100 µm ganz besonders bevorzugt eingesetzt.

Für das Verfahren sind Sirupe von beispielsweise Glucose, Mannose, Galaktose, Gulose, Allose oder Talose einsetzbar. Vorzugsweise wird dabei ein Glucosesirup verwendet. Der Sirup kann 10 bis 70 % Wasser enthalten. Wassergehalte von 10 bis 40 % werden dabei bevorzugt.

Zur Charakterisierung der Monosaccharide wird der Gehalt an reduzierenden Zuckern, berechnet als Dextrose auf der Basis der Trockensubstanz, bestimmt und als Dextroseäquivalent bezeichnet. Während demnach ein reines Monosaccharid ein Dextroseäquivalent von 100 aufweist, zeigen Dextroseäquivalente unter 100 einen Anteil an Oligosacchariden an. Erfindungsgemäß liegt das Dextroseäquivalent vorzugsweise bei 93 bis 95.

Bei der zweistufigen Herstellung werden zur Glycosidierung C₂- bis C₆-Alkohole, wie beispielsweise Ethanol, n-Propanol, Isopropanol, n-Butanol, t-Butanol, Amylalkohol oder Hexanol, eingesetzt.

Monosaccharid und C₂- bis C₆-Alkohol werden dabei vorzugsweise im molaren Verhältnis von 1 : 5 bis 1 : 10 zur Reaktion gebracht.

Die bei der Glycosidierung hergestellten Alkylglycoside mit C₂- bis C₆-Alkylketten werden in einer zweiten Stufe, der Umglycosidierung, mit C₈-bis C₂₀-Fettalkoholen umgesetzt. Die Fettalkohole können dabei linear oder verzweigt sein. Sie können auch olefinische Doppelbindungen enthalten. Man kann natürliche oder synthetische Fettalkohole oder Fettalkoholgemische einsetzen. Als Beispiele seien Octanol, Decanol, 10-Undecen-1-ol, Dodecanol, Myristylalkohol und Stearylalkohol genannt. Vorzugsweise werden Fettalkohole mit 10 bis 16 C-Atomen eingesetzt. Meist wird das Alkylglycosid mit C₂- bis C₆-Alkylgruppen im Molverhältnis von 1 : 4 bis 1 : 10 mit dem Fettalkohol umgesetzt.

Bei der Direktglycosidierung werden die gleichen Saccharide und Fettalkohole wie bei der zweistufigen Herstellung verwendet. Das Saccharid-Fettalkohol-Molverhältnis liegt dabei meist im Bereich von 1 : 4 bis 1 : 10. Vorzugsweise werden durch Direktglycosidierung Alkylglycoside mit C₈- bis C₁₄-Alkylresten hergestellt.

Bei der ein- und zweistufigen Herstellung kann man als saure Katalysatoren Mineralsäuren, wie Schwefel- oder Salzsäure, einsetzen. Gut geeignet sind auch organische Säuren, wie beispielsweise Aryl-, Alkyl- oder Aralkylsulfonsäuren. Dabei liegt die Reaktionstemperatur im allgemeinen im Bereich von 100 bis 140 °C. Die Glycosidierung führt man vorzugsweise bei 100 bis 125 °C durch. Bei der Umglycosidierung und bei der Direktglycosidierung liegt die Reaktionstemperatur meist bei 105 bis 135 °C, wobei zur Beschleunigung der Reaktion im allgemeinen Vakuum angelegt wird.

Die ein- und zweistufige Herstellung der Alkylglycoside kann kontinuierlich oder diskontinuierlich erfolgen. Dabei wird die kontinuierliche Alkylglycosid-Synthese üblicherweise in einer Rührkesselkaskade durchgeführt, wobei die Rührkessel auch teilweise oder vollständig durch Reaktionskolonnen oder Rohrreaktoren ersetzt werden können.

Bei der zweistufigen Alkylglycosid-Herstellung wird vorzugsweise nach jeder Stufe filtriert. Bei der Direktglycosidierung wird vorzugsweise nur nach der Reaktion eine Filtration vorgenommen. Bei Kaskaden von Reaktoren kann in den Leitungen zwischen den Reaktoren und bei Reaktoren mit Umläufen kann auch in diesen Umläufen filtriert werden.

Das am Ende der Reaktion anfallende, in Fettalkohol gelöste Alkylglycosid wird in bekannter Art und Weise mit geeigneten Basen neutralisiert. Der überschüssige Fettalkohol wird dann destillativ abgetrennt, was in einem Dünnschicht- oder Kurzwegverdampfer erfolgen kann. Das gewonnene Alkylglycosid, das vorzugsweise einen mittleren Glycosidierungsgrad von 1,1 bis 1,3 aufweist, wird dann mit Wasser abgemischt und mit Peroxid gebleicht. Das Endprodukt weist eine Iodfarbzahl (IFZ) von unter 20 und vorzugsweise einen Restfettalkoholgehalt von unter 1 % auf.

Das erfindungsgemäße Verfahren führt zu Reaktionsmischungen mit einem sehr geringen Anteil an ungelösten Bestandteilen. Es kommt nicht zu Vercrackungen, die die Farbe des Produktes beeinträchtigen könnten. Rohrleitungen und Reaktoren neigen nicht zu Verstopfungen. Das erhöht die Betriebssicherheit und die Standzeit der Reaktoren. Gleichzeitig werden direkt Alkylglycoside mit geringem Glycosidierungsgrad erhalten, wie sie mit Vorteilen in Wasch- und Geschirrspülmitteln eingesetzt werden.

## Patentansprüche

1. Verfahren zur ein- oder zweistufigen Herstellung von Alkylglycosiden mit C₈- bis C₂₀-Alkylgruppen und einem mittleren Glycosidierungsgrad zwischen 1,05 und 1,4,
dadurch gekennzeichnet,
daß man einen Saccharidsirup mit einem Dextroseäquivalent von 90 bis 95 einsetzt und Oligosaccharid während und/oder nach der Herstellung abfiltriert.

2. Verfahren nach Anspruch 1,
dadurch gekennzeichnet,
daß man beim Abfiltrieren Filter mit einer Porengröße von 50 bis 200 µm einsetzt.

3. Verfahren nach Anspruch 2,
dadurch gekennzeichnet,
daß Filter der Porengröße von 80 bis 100 µm verwendet werden.

4. Verfahren nach Anspruch 1,
dadurch gekennzeichnet,
daß als Saccharidsirup ein Glucosesirup eingesetzt wird.

5. Verfahren nach Anspruch 1,
dadurch gekennzeichnet,
daß Alkylglycoside mit einem mittleren Glycosidierungsgrad von 1,1 bis 1,3 hergestellt werden.
